# EUROPEAN PATENT SPECIFICATION

(11) **EP 0 776 607 B1**
(45) Date of publication and mention of the grant of the patent: **23.01.2002**
(21) Application number: 95203394.2
(22) Date of filing: 07.12.1995
(51) Int. Cl.: A23F 5/18, C07D 473/12

(54) **Caffeine imprint polymer**
Durch Coffein molekular geprägte Polymere
Polymères modelés de manière moléculaire par la cafféine

(43) Date of publication of application: 04.06.1997
(73) Proprietor: SOCIETE DES PRODUITS NESTLE S.A., 1800 Vevey (CH)
(72) Inventor: Hay, Philip, Macclesfield SK11 8BL (GB); Leigh, David, Manchester M15 6HR (GB); Liardon, Rémy, CH-1010 Lausanne (CH)

(56) References cited:
- WO-A-92/13447
- US-A- 4 364 964
- HIGHLY SEL. SEP. BIOTECHNOL., 1994, pages 207-225, XP002004763 L.I.ANDERSSON ET AL.: "Molecular imprinting - a versatile technique for the preparation of separation materials of predetermined selectivity"
- CHEM. LETT., vol. 10, 1995, pages 927-928, XP002004764 TAKAOMI KOBAYASHI ET AL.: "Molecular Imprinting of Theophylline in Acrylonitrile-acrylic Acid Copolymer Membrane"

## Description

The present invention relates to a new caffeine imprint polymer suitable for food application which selectively remove caffeine from a coffee extract, a process to decaffeinate a coffee extract, and a cartridge containing a caffeine imprint polymer.

### Background of the invention

It is well known that there is currently a popular demand for decaffeinated coffee and that it is enjoying a growing popularity for medical or dietetic reasons or more simply for reasons connected with lifestyle. Consequently, the food industry has developed many techniques to decaffeinate large quantities of coffee extract.

Thus, EP0040712 (Société des Produits Nestlé S.A.) describes a process for the recovery of caffeine from an aqueous solution of caffeine extracted from green coffee beans and non-caffeine green coffee solids, which comprises contacting the solution with activated carbon and separating the activated carbon, with caffeine adsorbed thereon, from aqueous solution of reduced caffeine content, characterised in that the activated carbon used is a neutralised carbon so that when it is immersed in water the pH value of the water is substantially unchanged.

Likewise, EP666033 (Kraft Foods, Inc.) describes a process for decaffeinating caffeine-containing aqueous extracts by which caffeine is adsorbed from the liquid onto activated carbon fibers.

However, although activated carbon such as charcoal is readily available and easily regenerated, it has been observed that activated carbon removes considerable quantities of non caffeine solids and some flavours. As it is virtually impossible to recover these solids and flavours without entraining caffeine, these materials cannot be reincorporated into the decaffeinated coffee. Their loss is a disadvantage from an economical point of view as well as with respect to the flavour quality of the decaffeinated coffee.

To avoid the disadvantage of using active carbon, EP0049357 (Société des Produits Nestlé S.A.) proposes a process for extracting caffeine from an aqueous solution containing it together with other non-caffeine solids, the aqueous solution having been derived from green coffee, characterised in that the solution is contacted with a non-ionic microporous resin having pores with an average diameter of 60 to 400 nm and preferably a surface area of more than 400 m²/g, whereby caffeine is extracted from said solution.

In other technical fields, imprint polymers have been found to be effective means to selectively remove molecules from a chemical or pharmaceutical bulk material. The procedure generally involves incorporation of small amounts of an imprint molecule in a polymerisation medium. The imprinting molecule is removed after polymerization, leaving a functionalized cavity in the macromolecular network.

These imprint polymers have been up to the present time mostly used in the laboratory, for instance as stationary phases in a high performance liquid chromatographic (HPLC) mode for enantiomeric separation of amino acid derivatives (B. Sellergren et al., Chromatography, 17, 994-998, 1985).

Recently, theophylline and diazepam imprint polymers have been also used as synthetic antibodies substitutes to make competitive binding assays for theophylline and diazepam determination in human serum. The method relies on the inhibition of radiolabelled ligand (theophylline or diapezam) binding by the serum. The amount of labelled ligand bound to the polymer is inversely related to the concentration of drug present in the sample (G. Vlatakis et al., Nature, 361, 645-647, 1993).

However, it can be noticed that imprint polymers have been used on a laboratory scale to specifically separate, retain or remove a molecule from a diluted solution, at room temperature. It has never been envisaged that these polymers could also be used in an industrial scale, especially for food purposes, to specifically remove caffeine from a complex extract containing other non-caffeine solids at high temperatures.

Imprint polymers used for separating therapeutic agents are e.g. described in Highly Sel. Sep. Biotechnol. 94, 1994, pp. 207-225, Chem. Lett. 10, 1995, pp. 927-928, WO-A-9213447, and US 4,364, 964.

### Summary of the invention

The present invention avoids the disadvantages described above and is an alternative to active carbon or known resins used to decaffeinate a coffee extract.

Thus, the invention provides a use of a caffeine imprint polymer to decaffeinate coffee extract.

It is preferred that the imprint polymer has non covalent recognition sites for caffeine, which when in contact with an aqueous extract derived from green or roasted coffee be removes removes . Preferably the caffeine imprint polymer is formed by polymerizing a monomer in the presence of caffeine, the mass ratio of monomer (m) and caffeine (c) being in the range (m:c) of 0.1:1 to 100:1. Moreover, a cross-linking agent can be used in a mass ratio of monomer (m) to cross-linking agent (a) in the range of (m:a) 1:1 to 1:100.

According to another aspect of the invention, there is provided a process for extracting caffeine from an aqueous extract derived from green or roasted coffee beans, in which the solution is contacted with an imprint polymer having non covalent recognition sites for caffeine and capable to selectively remove caffeine from an aqueous extract, whereby caffeine is removed from said extract.

The invention also relates to the use of a imprint polymer, contained in a cartridge, to decaffeinate a coffee extract from roased coffee beans, the imprint polymer having non covalent recognition sites for caffeine.

Thus, it has been possible to develop new imprint polymers capable to specifically remove from an aqueous coffee extract more than 99% of free caffeine, without any special difficulties or complications. Surprisingly, it has been also shown that coffee flavours are not substantially removed from the coffee extract.

Imprint polymers according to the present invention can therefore be used advantageously in an industrial process to remove caffeine from an aqueous solution derived from coffee green beans. They can be also used in a cartridge to remove caffeine easily from a coffee extract or a coffee brew destined normally to be directly consumed in a cup.

The invention has the advantage that the imprint polymer can also be periodically regenerated which allows its extensive use.

Also, the invention has the advantage that the imprint polymer does not release any starting materials such as monomers and cross-linking agents.

For purposes of this disclosure and claims, "caffeine imprint polymer" or "imprint polymer" or even "polymer" is intended to mean a polymer comprising functionalized cavities in a macromolecular network which are specific for the three-dimensional structure of caffeine's molecule by allowing stable non-covalent bounds.

### Detailed description of the invention

The preparation of the polymer according to invention involves incorporation of amounts of caffeine in a medium comprising monomers and cross-linking agents, polymerization of the medium, and removal of caffeine molecules which liberates the functionalized cavities.

To this end, the mass ratio of monomers (m) and caffeine (c) used in the medium may be comprised in a range (m:c) of 0.1:1 to 100:1, preferably 0.5:1 to 50:1, and the mass ratio of cross-linking agents (a) and monomers (m) used in the medium may be comprised in a range (m:a) of 1:1 to 1:100, preferably 1:2 to 1:50, for example. The solvent used to dissolve the different components is preferably an organic solvent such as chloroform, for example.

The mixture may be then cooled down to -5°C to +5°C, degassed, for example under vacuum in a sonicating water bath, and purged with nitrogen for 1-60 min, preferably 3-10 min. The polymerization may be afterwards induced at 40-80°C during 10-48 h by a polymerization agent such as 2,2'-azobis(2-methylpropionitrile) (AIBN), the mass ratio of polymerization agent (p) and monomers (m) being preferably in a range (p:m) of 0.1:1 to 10:1. The polymerization may be also induced by an U.V. source at -5°C to +5°C during 10-48 h, for example.

The bulk polymers may be ground, for instance in a mechanical mortar, sieved through a 10-500µm sieve, sedimented several times to remove any fines, and extracted by extensive washing with an elution solution preferably selected among acidic organic solvents such as methanol (e) comprising acetic acid (i) in a volume ratio (e:i) of 5:1 to 15:1. This extraction step allows removal of caffeine, and unreacted monomers and cross-linking agents. Any residual solvent may be afterwards removed by extensive washing with water. Finally, the polymer particles may be dried under vacuum, for example.

Preferably, dried particles of caffeine imprint polymer have a volumetric mass of at least 10 g/cm³.

The monomer used for the macromolecular network may be any molecule able to form at least intermolecular hydrogen bonds with caffeine. Preferably, it is selected, alone or in combination, from chlorogenic acid, gallic acid, methacrylic acid and the molecule having the formula 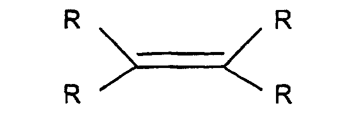 in which at least one radical is a carboxyl or an amide, and the others are selected from hydrogen, aldehyde, alkyl, alkenyl, alkoxyl, alkynyl, amine, amide, aryl, arylketoyl, aryloxy, bromide, carbonyl, carboxyl, chloride, cycloalkyl, fluoride, hydroxyalkyl, hydroxyaryl, hydroxyl, iodide, phenoxy, phenol, phenyl, polyunsaturated carbon open chain, polyunsaturated carbon ring, nitrogen-, oxygen-, and/or sulfur- containing compounds, containing for example heterocyclic, polyhydroxy alkyl and polyhydroxyaryl moieties.

Particularly, the monomer having the above formula comprises one -CO₂H or -CONH2 radical, the other radicals being selected from -H, -CH₃, -CH₂-CH₃, - CO₂H, -CH₂-CHO, -CONH₂, -Phenyl-CHO, Phenyl-(OH)₂ and -Cl. The preferred monomer of the above formula is caffeic acid.

Preferably, cross-linking agents used to make the imprint polymer are any known cross-linking agents which would not form a covalent bound with caffeine and which would form a rigid backbone, such as glutaraldehyde, bisimidate and N-hydroxysuccinidate, for example. However, it is desirable to use low hydrophobic cross-linking agents such as 2-hydroxy-ethylene-glycol (HEG) and ethylene-glycol-dimetacrylate (EDMA), alone or in a mixture thereof.

In another aspect of the invention, the caffeine imprint polymer particles described above may be used in a process for removing caffeine from an aqueous extract derived from green or roasted coffee beans.

Thus, an aqueous coffee extract may be obtained by conventional methods, which involves contacting green coffee beans with water for a period of time sufficient to reduce the caffeine content of the beans to a desired level, or simply passing hot water through a bed of ground roasted coffee beans containing caffeine, for example.

In one preferred embodiment, a green coffee extract is thus contacted with caffeine imprint polymer. The contacting may be effected in a counter-current system, using an arrangement similar to those used for extracting roasted coffee in the preparation of coffee extract, or batch may be employed wherein a fixed volume of water is continuously recycled to a fixed weight of coffee beans, caffeine being removed from the extract at each cycle prior to its return to the beans. In the counter-current system, the aqueous extraction medium containing caffeine and non-caffeine green solids encounters coffee of progressively higher caffeine content.

The main factors affecting operations are temperature, the ratio of extraction liquid to coffee, the ratio of imprint polymer to coffee, time and liquid velocity, each of which may be adapted to the degree of decaffeination desired, which is preferably such that the caffeine content is substantially reduced to zero.

More in detail, green coffee beans may be contacted as a static bed, in a column or in a suitable tumbler or like extractor. In both cases, the weights of coffee and water are constant, with the caffeine-laden water withdrawn from the column or extractor being decaffeinated with imprint polymer prior to its being recycled. The weight of imprint polymer may be 10 to 50% of the weight of green coffee being decaffeinated. The total contact time will depend on the water/coffee ratio, temperature and the degree of decaffeination desired. Usually, it is 3 to 20 hours, for example. The temperature is preferably in the range 60 to 90°C. Lower temperatures are usually avoided as the risk of microbial growth, especially with long contact times, is increased, resulting in fermentation of the sugars present in the extract. In addition, the rate of caffeine diffusion from the beans decreases with decreasing temperature. Above 90°C, with long contact times, flavour may be impaired, and as a practical matter it is difficult to maintain these temperatures without resort to pressurised equipment.

On the other hand, green coffee beans may be also continuously extracted and decaffeinated in a continuous counter-current system using an arrangement similar to those used for extracting roasted coffee. The number of extractors and cycle time are chosen to give the desired degree of decaffeination.

Decaffeination of green coffee beans is preferably effected with deionised water. The water to coffee ratio is by no means critical, but rather is determined having regard to practical considerations imposed by industrial operations. Excessive volumes are thus avoided, as also water to coffee ratios which do not provide for adequate caffeine removal. It has been found that in general a weight ratio of water to green coffee of at least about 3 parts of water per part of coffee gives satisfactory results, preferably 3 to 100 parts.

When decaffeination is terminated, it is usually desirable to return the non-caffeine solids present in the aqueous extract to the decaffeinated green beans. Various techniques may be used. For example, the beans may be pre-dried to 10-45% by weight moisture and combined directly with the decaffeinated extract. Alternatively, the decaffeinated extract may be pre-concentrated to a solids content of 15-55% by weight before contact with the beans. Desirably, the total amount of water present is such that the final moisture content of the beans does not exceed about 55% by weight. After reincorporation of solids the coffee beans may be dried to a moisture content of 5-12% by weight before being roasted.

The process according to the invention is more favourable from the economic point of view if the imprint polymer can be regenerated, i.e. freed from the caffeine and non-caffeine solids which it removes. It may then be used again in a subsequent operation. For regeneration, polar acidic solvents are conveniently used, such as methanol:acetic acid (9:1) for example.

In another embodiment of the invention, the imprint polymer according to the present invention can be used also to remove caffeine easily from an extract or infusion derived from ground roasted beans. To this end, the present invention provides a cartridge comprising a bed of caffeine imprint polymer, through which the consumer can pass a coffee extract or brew to remove caffeine. The cartridge may consist of a plastic or metal body formed by a cavity having a circular, oval or polygonal shape, for example, containing particles of imprint polymer being closed by a membrane. An amount as low as 50 g of imprint particles may be sufficient to decaffeinate substantially 1 litre of a coffee brew, for example.

These cartridges can be made in an open form with permeable upper and lower faces to allow the extract to pass through. In order to maintain the quality of the imprint polymer by protecting it against bacterial contamination, for example, open cartridges have to be wrapped in fluid-type bags or provided with fluid-tight peelable covers on both faces. The cartridge preferably has a diameter about 2 to about 20 cm, preferably 5 to 10 cm, and a length of about 0.5 to 20 cm, preferably 1 to 5 cm.

The invention is illustrated by the following examples, in which all parts, ratios, and percentages are expressed on a weight basis unless otherwise stated, with reference to the accompanying drawings in which:

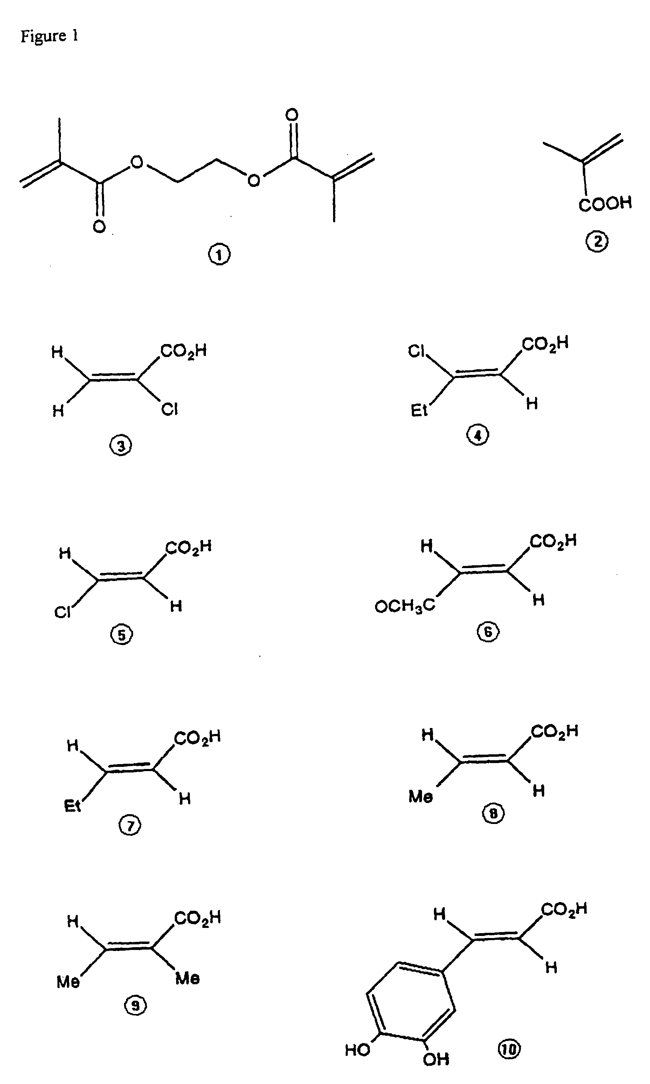

- Fig. 1 presents the structure of EDMA cross-linking agent (n°1), and a list of different monomer structures (n°2 to 10);

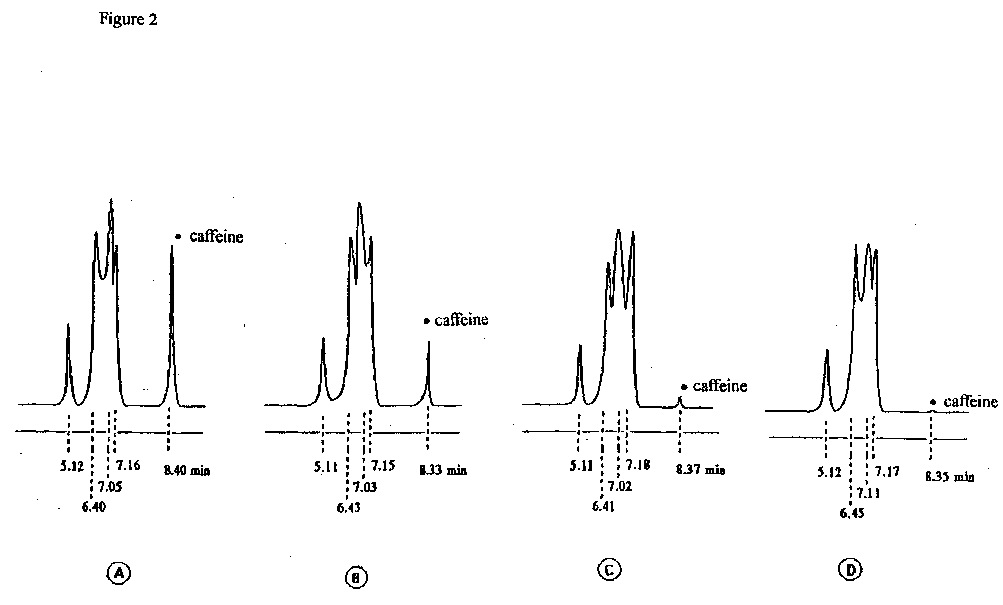

- Fig. 2 presents four HPLC profiles relating to decaffeination of instant coffee by a caffeine imprint polymer made with methacrylic acid (monomer no 2) and EDMA, profile A being related to natural instant coffee (no decaffeination), and profiles B, C, D being related respectively to instant coffee being more than 50%, 90% and 99% decaffeinated;

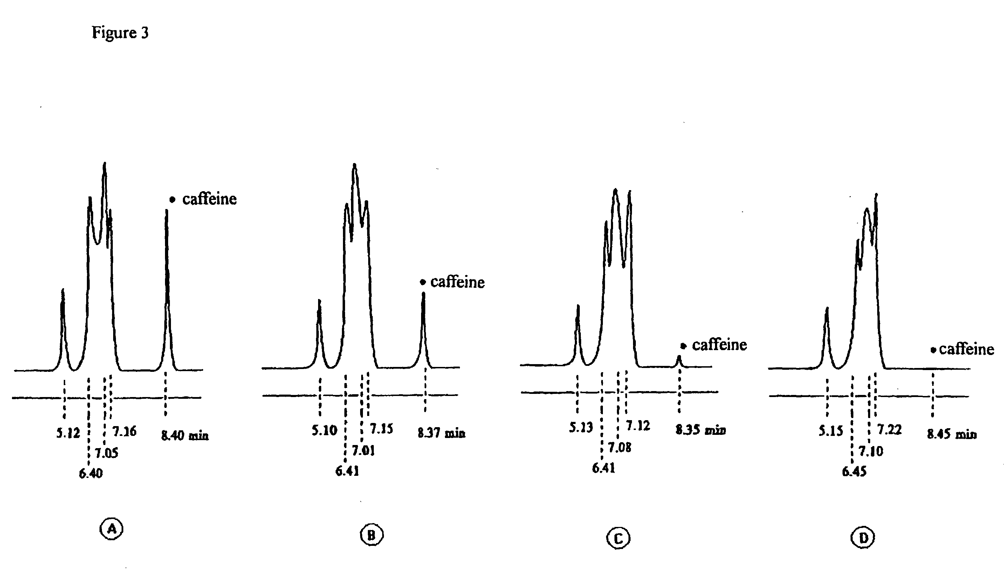

- Fig. 3 presents four HPLC profiles relating to decaffeination of instant coffee by a caffeine imprint polymer made with 2-chloropropenoic acid (monomer no 3) and EDMA, profile A being related to natural instant coffee (no decaffeination), and profiles B, C, D being related respectively to instant coffee being more than 50%, 90% and 99% decaffeinated;

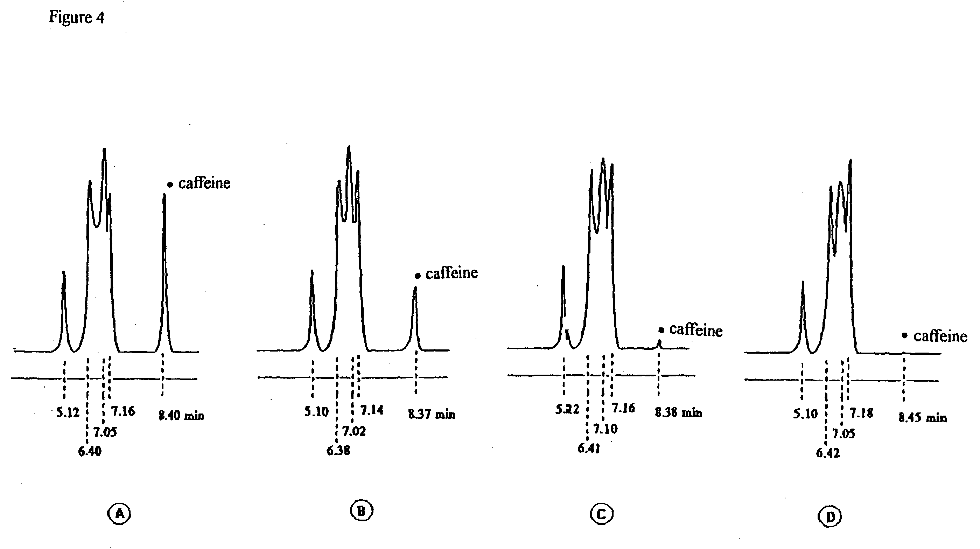

- Fig. 4 presents four HPLC profiles relating to decaffeination of instant coffee by a caffeine imprint polymer made with caffeic acid (monomer no 10) and EDMA, profile A being related to natural instant coffee (no decaffeination), and profiles B, C, D being related respectively to instant coffee being more than 50%, 90% and 99% decaffeinated;

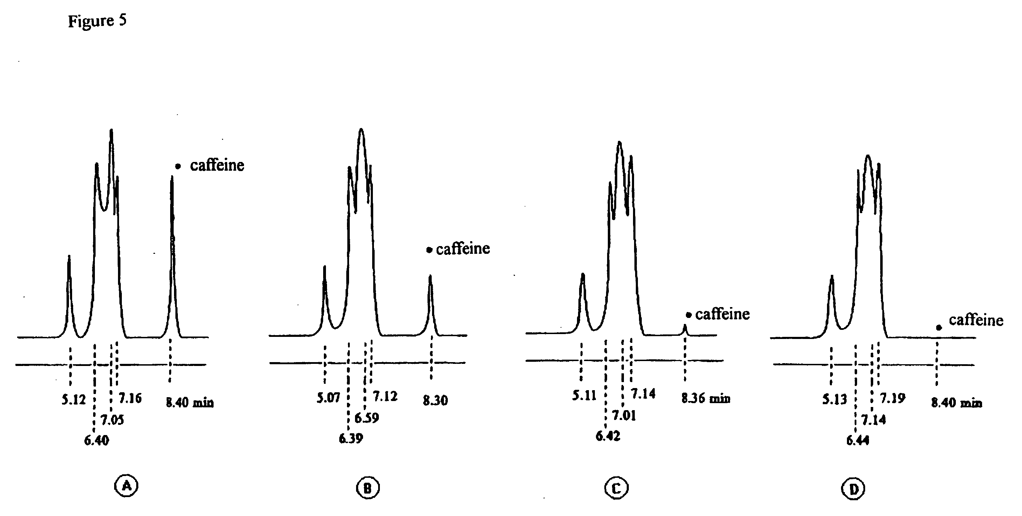

- Fig. 5 presents four HPLC profiles relating to decaffeination of instant coffee by a caffeine imprint polymer made with caffeic acid, methacrylic acid and EDMA, profile A being related to natural instant coffee (no decaffeination), and profiles B, C, D being related respectively to instant coffee being more than 50%, 90% and 99% decaffeinated.

### Examples 1-9

A solution in chloroform of 1 part of a monomer (fig. 1, monomers 2-10), 10 parts of EDMA, 1 part of caffeine and 1 part of AIBN is prepared. The solution is degassed in a sonicating water-bath and purged with nitrogen. Polymerization takes place during heating at 60°C for 24h. The bulk polymer is then ground to a fine powder and sedimented in acetonitrile to remove any fines. The print molecule is then extracted by extensive washing with a solution of methanol/acetic acid (9/1, v/v). Any residual solvent is afterwards removed by extensive washing with water. The imprint polymer particles are finally dried under vacuum.

Each imprint polymer is then used to decaffeinate instant coffee. To this end, 100 mg of Nescafé gold blend® is dissolved in 100 ml water and passed through a 20mm diameter column packed with a quantity of an imprint polymer. The amount of caffeine remaining in the sample is determined by HPLC. The quantity of polymer is varied up to a maximum of 35g until the integration of the caffeine signal is respectively lower than 50%, 10% and 1% of the initial quantity present in the sample.

Conditions used for HPLC are chosen to get good separation between the caffeine peak and the other components of the sample. The Wavelength of detection is also chosen to minimise interference from trace components close to the caffeine signal. HPLC is thus performed in reverse phase with a LiCrosorb® RP 8 column (company, country), 50cm x 4 mm, at 190 bar under isocratic conditions with 2% acetonitrile in water (v/v), at a flow rate of 3 ml/min and with a detection at 254nm.

For comparison, an HPLC is also performed using directly 100 mg of Nescafé gold blend® dissolved in 100 ml water.

The results presented in table 1 below, show that it is possible to achieve more than 99% of decaffeination with as little as 12% by weight of imprint polymer per volume of a traditional coffee brew. Indeed, the HPLC profiles relating to decaffeination operated by imprint polymer of example 1, 2 and 9 (see figure 2-4), show clearly that more than 99% of caffeine can be removed from the extract.

Each decaffeinated brew is described by a panel of trained tasters as being of good flavour and colour, similar to those of the initial extract.

Moreover, It must be noted that these polymers do not remove a solution of 5% theophylline. Thus, these polymers are only specific for caffeine molecules. The best monomers are methacrylic acid (ex. 1), 2-chloropropenoic acid (ex. 2) and caffeic acid (ex.9).

**Table 1**

| Example | Monomer (see n° of monomer structure in figure 1) | Amount of polymer to remove 50% of caffeine (g) | Amount of polymer to remove 90% of caffeine (g) | Amount of polymer to remove 99% of caffeine (g) |
|---|---|---|---|---|
| 1 | n° 2 | 3.9 | 11.0 | 15.8 |
| 2 | n° 3 | 3.5 | 8.3 | 13.5 |
| 3 | n° 4 | 4.7 | 18.8 | 25.6 |
| 4 | n° 5 | 4.6 | 17.3 | 23.3 |
| 5 | n° 6 | 5.2 | 18.9 | 28.0 |
| 6 | n° 7 | 5.8 | 20.1 | >35 |
| 7 | n° 8 | 5.3 | 19.2 | 33.0 |
| 8 | n° 9 | 6.6 | 22.7 | >35 |
| 9 | n° 10 | 2.8 | 5.9 | 12.0 |

### Example 10

A solution in chloroform of methacrylic acid (4.5g), EDMA (45g), caffeic acid (9g), caffeine (5g) and AIBN (1g) is prepared. The solution is degassed in a sonicating water-bath and purged with nitrogen for 10 min. Polymerization takes place during heating at 60°C for 24h. The solvent is decanted off, and the bulk polymer is then ground to a fine powder and sedimented twice in acetonitrile to remove ultrafine particles. The print molecule is then extracted by extensive washing with a solution of methanol/acetic acid (9/1, v/v). Any residual solvent is afterwards removed by extensive washing with water until the aqueous washings are pH neutral. The imprint polymer particles are finally dried under vacuum at 60°C for 4h.

Imprint polymer is then washed several times with boiling water during 24h. The aqueous washings are analysed by Infra Red absorbtion (I.R.), the spectrum chosen being 3700-3020, 2950, 2580, 1920, 1730, 1700-1000 cm⁻¹. Results show that no material is leached from the polymer under these conditions.

### Examples 11-12

Imprint polymers made from a mixture of EDMA and different ratio of caffeic acid (CA) and methacrylic acid (MAA), are prepared in a manner similar to that described in example 10. Each imprint polymer is then used to decaffeinate instant coffee according to the procedure described in examples 1-9. The amount of caffeine remaining in the sample is also determined by HPLC with the same conditions. The quantity of polymer is varied up to a maximum of 35g until the integration of the caffeine signal is respectively lower than 50%, 10% and 1% of the initial quantity present in the sample.

The results presented in table 2 below, show that it is possible to achieve more than 99% of decaffeination with as little as 5.5% by weight of imprint polymer per volume of a coffee extract or a coffee brew. The HPLC profiles relating to decaffeination operated by imprint polymer of example 12 (see figure 5), show clearly that more than 99% of caffeine can be removed from the extract or brew. A sensorial test shows also that each decaffeinated extract presents a colour and flavour similar to those of the initial extract or brew.

**Table 2**

| Ex. | Polymer ratio of CA: MAA | Amount of polymer to remove 50% of caffeine (g) | Amount of polymer to remove 90% of caffeine (g) | Amount of polymer to remove 99% of caffeine (g) |
|---|---|---|---|---|
| 1 | 0:100 | 3.9 | 11.0 | 15.8 |
| 11 | 25:75 | 3.3 | 8.0 | 12.4 |
| 12 | 50:50 | 1.2 | 3.5 | 5.5 |
| 10 | 75:25 | 1.8 | 5.3 | 7.9 |
| 9 | 100:0 | 2.8 | 5.9 | 12.0 |

### Example 13

Green coffee extract is prepared by grinding green coffee beans (50g) to a fine powder with a commercial coffee grinder. The grounds were extracted with distilled water (500ml) for 60 min at 90°C. The liquid phase is separated by filtration and the filtrate concentrated by reducing to half the original volume under reduced pressure.

50 ml of this solution is passed through a 20 mm diameter column packed with a quantity of an imprint polymer of examples 1-9 and 12. The amount of caffeine remaining in the sample is determined by HPLC in the same conditions. The quantity of polymer is varied up to a maximum of 35g until the integration of the caffeine signal is lower than 1% of the initial quantity present in the sample.

The results presented in table 3 below show that 12.3% of an imprint polymer per volume of green coffee extract is sufficient to remove 99% of the caffeine from the extract.

**Table 3**

| Polymer of example | 1 | 2 | 3 | 4 | 5 | 6 | 7 | 8 | 9 | 12 |
|---|---|---|---|---|---|---|---|---|---|---|
| Amount of polymer to remove > 99% caffeine (g) | 17.3 | 15.6 | 30.0 | 25.8 | 30.0 | >35 | >35 | >35 | 14.0 | 12.3 |

### Examples 14-16

Imprint polymers made with different ratio of caffeine, EDMA and methacrylate acid, are prepared in a manner similar to that described in examples 1-9.

Each imprint polymer is then used to remove caffeine from a solution of 1.03x10⁻³ mol/l caffeine (50mg in 250 ml water). After the solution has been treated with polymer in a batch, the water is removed, the particles are dried and afterwards weighed. The imprint polymers presented in table 4 below remove all 98% of caffeine. Consequently, changing the component ratio does not seem to have beneficial effects on caffeine adsorption.

**Table 4**

| Polymer's example | Caffeine: MAA: EDMA ratio (part) |
|---|---|
| 1 | 1:1:10 |
| 14 | 1:1:5 |
| 15 | 6:1:6 |
| 16 | 1:6:60 |

### Example 15

Imprint polymer is prepared using MAA (1 part), caffeine (1 part), HEG (5 part) and EDMA (5 part) in a manner similar to that described in examples 1-9. This polymer has similar properties as those described for the polymer of example 1.

### Example 16

Green coffee beans are decaffeinated continuously by counter-current extraction with an aqueous solution. To this end, the process described in example 5 of EP0040712 is carried out with the exception that activated carbon is replaced by the imprint polymer of example 12. Finally, the decaffeinated green beans are dried to 20% moisture and mixed with the corresponding quantity of decaffeinated extract, which has first been concentrated to 20% solids. Mixing is continued for 6 hours at 65°C. Thereafter, the coffee containing 45% moisture is dried to 8.5% moisture content. The decaffeinated coffee has a good appearance, similar to the original non-decaffeinated beans. When roasted and prepared as an infusion, the brew is described by a panel of trained tasters as being of good quality and colour.

### Example 17

Roasted non-decaffeinated beans are ground, an infusion is prepared, and 1 litre of the brew is decaffeinated using a cartridge having permeable upper and lower faces and containing 100 g of imprint polymer of example 12, closed with a membrane.

The brew is described by a panel of trained tasters as being of good quality and colour. When milk is added to the cup, the brew assumes a red-brown colour, without traces of greyness.

## Claims

1. Use of a caffeine imprint polymer to decaffeinate coffee extract.

2. Use of an imprint polymer as claimed in claim 1, having non covalent recognition sites for caffeine, which when in contact with an aqueous extract derived from green or roasted coffee beans selectively removes caffeine.

3. Use of an imprint polymer as claimed in either claim 1 or claim 2, which is formed by polymerizing a monomer in the presence of caffeine, the mass ratio of monomer (m) and caffeine (c) being in the range (m:c) of 0.1:1 to 100:1.

4. Use of an imprint polymer as claimed in claim 3, in which a cross-linking agent is used in a mass ratio of monomer (m) to cross-linking agent (a) in the range of (m:a) 1:1 to 1:100.

5. Use of an imprint polymer as claimed in either of claims 3 and 4, in which the monomer is selected, alone or in combination, from chlorogenic acid, gallic acid, methacrylic acid and the molecule having the formula 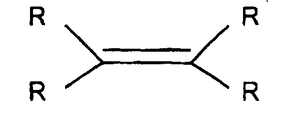 in which at least one radical is a carboxyl or an amide, and the others are selected from hydrogen, aldehyde, alkyl, alkenyl, alkoxyl, alkynyl, amine, amide, aryl, arylketoyl, aryloxy, bromide, carbonyl, carboxyl, chloride, cycloalkyl, fluoride, hydroxyalkyl, hydroxyaryl, hydroxyl, iodide, phenoxy, phenol, phenyl, polyunsaturated carbon open chain, polyunsaturated carbon ring, nitrogen-, oxygen-, and/or sulfur- containing compounds.

6. Use of an imprint polymer as claimed in claim 5, in which the monomer has the formula 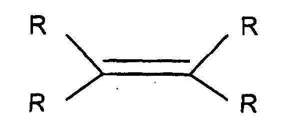 in which one radical is -CO₂H or -CONH2, and the other radicals are selected from -H, -CH₃, -CH₂-CH₃, -CO₂H, -CH₂-CHO, -CONH₂, -Phenyl-CHO, Phenyl-(OH)₂ and -Cl.

7. Use of an imprint polymer as claimed in claim 6, in which the monomer is caffeic acid.

8. Use of an imprint polymer as claimed in either claim 1 or claim 2, in which the cross-linking agent used to make the imprint polymer is a low-hydrophobic agent, more particularly 2-hydroxy-ethylene-glycol and ethylene-glycol-dimetacrylate, alone or in a mixture thereof.

9. Use of an imprint polymer contained in a cartridge to decaffeinate a coffee extract from roased coffee beans, the imprint polymer having non covalent recognition sites for caffeine.

10. A process for extracting caffeine from an aqueous extract derived from green or roasted coffee beans, in which the extract is contacted with an imprint polymer having non covalent recognition sites for caffeine and capable to selectively remove caffeine from an aqueous extract, whereby caffeine is removed from said extract.

## Patentansprüche

1. Verwendung eines durch Coffein molekular geprägten Polymers zur Entcoffeinierung eines Kaffeextrakts.

2. Verwendung eines geprägten Polymers nach Anspruch 1, das nichtkovalente Erkennungsstellen für Coffein aufweist, das dann, wenn es mit einem wäßrigen Extrakt auf der Basis von grünen oder gerösteten Kaffeebohnen in Kontakt kommt, selektiv Coffein entfernt.

3. Verwendung eines geprägten Polymers nach Anspruch 1 oder Anspruch 2, das durch Polymerisieren eines Monomers in Gegenwart von Coffein hergestellt wurde, wobei das Massenverhältnis von Monomer (m) und Coffein (c) im Bereich (m:c) von 0,1:1 bis 100:1 liegt.

4. Verwendung eines geprägten Polymers nach Anspruch 3, bei dem ein Vernetzungsmittel in einem Massenverhältnis von Monomer (m) zu Vernetzungsmittel (a) im Bereich (m:a) von 1:1 bis 1:100 verwendet wird.

5. Verwendung eines geprägten Polymers, wie es in einem von Anspruch 3 und Anspruch 4 beansprucht wird, wobei das Monomer einzeln oder in Kombination ausgewählt ist aus Chlorogensäure, Gallussäure, Methacrylsäure und dem Molekül mit der Formel 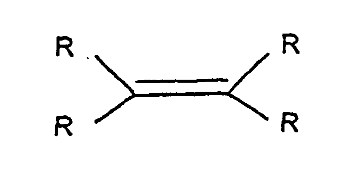 in der wenigstens ein Rest ein Carboxyl oder ein Amid ist und die anderen ausgewählt sind aus Wasserstoff, Aldehyd, Alkyl, Alkenyl, Alkoxyl, Alkinyl, Amin, Amid, Aryl, Arylketoyl, Aryloxy, Bromid, Carbonyl, Carboxyl, Chlorid, Cycloalkyl, Fluorid, Hydroxyalkyl, Hydroxyaryl, Hydroxyl, Iodid, Phenoxy, Phenol, Phenyl, offenen mehrfach ungesättigten Kohlenstoffketten, mehrfach ungesättigten Kohlenstoffringen, stickstoff-, sauerstoff- und/oder schwefelhaltigen Verbindungen.

6. Verwendung eines geprägten Polymers nach Anspruch 5, wobei das Monomer die Formel aufweist 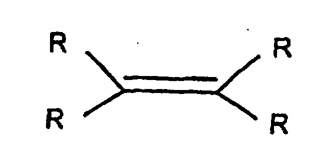 worin ein Rest -CO₂H oder -CONH₂ ist und die anderen Reste ausgewählt sind aus -H, -CH₃, -CH₂-CH₃, -CO₂H, -CH₂-CHO, -CONH₂, -Phenyl-CHO, Phenyl-(OH)₂ und -Cl.

7. Verwendung eines geprägten Polymers nach Anspruch 6, wobei das Monomer Kaffeesäure ist.

8. Verwendung eines geprägten Polymers nach entweder Anspruch 1 oder Anspruch 2, wobei das Vernetzungsmittel, das zur Herstellung des geprägten Polymers verwendet wird, ein Mittel mit niedriger Hydrophobie ist, insbesondere 2-Hydroxyethylenglycol und Ethylenglycoldimethacrylat, und zwar allein oder in einer Mischung daraus.

9. Verwendung eines geprägten Polymers, das in einer Patrone angeordnet ist, zur Entcoffeinierung eines Kaffeextrakts aus gerösteten Kaffeebohnen, wobei das geprägte Polymer nichtkovalente Wiedererkennungsstellen für Coffein aufweist.

10. Verfahren zum Extrahieren von Coffein aus einem wäßrigen Extrakt, der aus grünen oder gerösteten Kaffeebohnen erhalten wurde, wobei der Extrakt mit einem geprägten Polymer in Berührung gebracht wird, das nichtkovalente Wiedererkennungsstellen für Coffein aufweist und in der Lage ist, selektiv Coffein aus einem wäßrigen Extrakt zu entfernen, wodurch Coffein aus dem genannten Extrakt entfernt wird.

## Revendications

1. Utilisation d'un polymère à empreinte de caféine pour décaféiner un extrait de café.

2. Utilisation d'un polymère à empreinte suivant la revendication 1, ayant des sites de reconnaissance non-covalente pour la caféine qui, lorsqu'il est mis en contact avec un extrait aqueux dérivé de grains de café verts ou torréfiés, élimine sélectivement la caféine.

3. Utilisation d'un polymère à empreinte suivant la revendication 1 ou la revendication 2, qui est formé en polymérisant un monomère en présence de caféine, le rapport en masse de monomère (m) à la caféine (c) étant compris dans l'intervalle (m:c) de 0,1:1 à 100:1.

4. Utilisation d'un polymère à empreinte suivant la revendication 3, dans lequel un agent de réticulation est utilisé en un rapport en masse du monomère (m) à l'agent de réticulation (a) compris dans l'intervalle (m:a) 1:1 à 1:100.

5. Utilisation d'un polymère à empreinte suivant l'une des revendications 3 et 4, dans laquelle le monomère est choisi, seul ou en association, entre l'acide chlorogénique, l'acide gallique, l'acide méthacrylique et la molécule répondant à la formule : 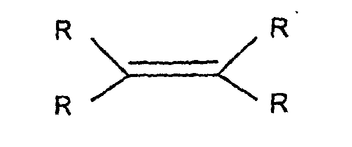 dans laquelle au moins un radical est un radical carboxyle ou un radical amide, et les autres radicaux sont choisis entre l'hydrogène, les radicaux aldéhyde, alkyle, alcényle, alkoxyle, alcynyle, amine, amide, aryle, arylcétoyle, aryloxy, bromure, carbonyle, carboxyle, chlorure, cycloalkyle, fluorure, hydroxyalkyle, hydroxyaryle, hydroxyle, iodure, phénoxy, phénol, phényle, une chaîne ouverte carbonée polyinsaturée, un noyau carboné polyinsaturé et des composés contenant de l'azote, de l'oxygène et/ou du soufre.

6. Utilisation d'un polymère à empreinte suivant la revendication 5, dans lequel le monomère répond à la formule : 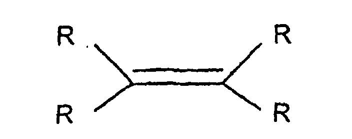 dans lequel un radical est un radical -CO₂H ou -CONH₂, les autres radicaux étant choisis entre -H, -CH₃, -CH₂CH₃, CO₂H, -CH₂-CHO, -CONH₂, -phényl-CHO, phényl-(OH)₂ et -Cl.

7. Utilisation d'un polymère à empreinte suivant la revendication 6, dans lequel le monomère est l'acide caféique.

8. Utilisation d'un polymère à empreinte suivant la revendication 1 ou la revendication 2, dans lequel l'agent de réticulation utilisé pour préparer le polymère à empreinte est un agent faiblement hydrophobe, plus particulièrement le 2-hydroxy-éthylèneglycol et le diméthacrylate d'éthylèneglycol, seuls ou sous forme de leurs mélanges.

9. Utilisation d'un polymère à empreinte logé dans une cartouche pour décaféiner un extrait de café obtenu à partir de grains de café torréfiés, le polymère à empreinte ayant des sites de reconnaissance non-covalente pour la caféine.

10. Procédé pour extraire la caféine d'un extrait aqueux café dérivé de grains de café verts ou torréfiés, dans lequel l'extrait est mis en contact avec un polymère à empreinte ayant des sites de reconnaissance non-covalente pour la caféine et capable d'éliminer sélectivement la caféine d'un extrait aqueux, la caféine étant ainsi éliminée dudit extrait.
